(19) **Europäisches Patentamt**
**European Patent Office**
**Office européen des brevets**

(11) **EP 3 709 308 A1**

(12) **EUROPEAN PATENT APPLICATION**

(43) Date of publication:
**16.09.2020 Bulletin 2020/38**

(51) Int Cl.:
***G16H 50/30*** *(2018.01)*  ***A61B 5/00*** *(2006.01)*

(21) Application number: **20162929.2**

(22) Date of filing: **13.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019 US 201962818836 P**

(71) Applicant: **Hill-Rom Services, Inc.**
**Batesville, IN 47006 (US)**

(72) Inventors:
 • **EMMONS, Kirsten**
  **Batesville, IN Indiana 47006 (US)**
 • **LIGHTCAP, Kristy Keaton**
  **Batesville, IA Iowa 47006 (US)**

 • **RECEVEUR, Timothy**
  **Batesville, IN Indiana 47006 (US)**
 • **RIORDAN, Matt**
  **Batesville, IN Indiana 47006 (US)**
 • **SHI, Yuan**
  **Batesville, IN Indiana 47006 (US)**
 • **URRUTIA, Eugene**
  **Batesville, IN Indiana 47006 (US)**
 • **CHUNG, Chiew Yuan**
  **Batesville, IN Indiana 47006 (US)**
 • **ZAPFE, Lori**
  **47006, IN Indiana (US)**

(74) Representative: **Loustalan, Paul William**
 **Reddie & Grose LLP**
 **The White Chapel Building**
 **10 Whitechapel High Street**
 **London E1 8QS (GB)**

(54) **PATIENT FALL LIKELIHOOD AND SEVERITY**

(57)      An example system for estimating an injury risk relating to a fall for a patient can include: at least one processor; and memory encoding instructions which, when executed by the at least one processor, cause the at least one processor to: access first data associated with an estimated likelihood of a fall for the patient; access second data associated with an estimated severity of a fall for the patient; and calculate a score based upon the first data and the second data.

FIG. 2

**EP 3 709 308 A1**

**Description**

INTRODUCTION

Patients in care facilities, such as hospitals, clinics, nursing homes or the like, are often in compromised medical conditions. Injuries sustained by patients in a care facilities result in significant healthcare costs. In an effort to prevent such injuries, various protocols are implemented to mitigate the risks. For example, patients who are at risk of falling when moving unassisted may be identified as fall risks, and certain protocols may be implemented to reduce the opportunity for the patients to move about the room unassisted.

SUMMARY

A system for estimating an injury risk relating to a fall for a patient, the system comprising: at least one processor; and memory encoding instructions which, when executed by the at least one processor, cause the at least one processor to: access first data associated with an estimated likelihood of a fall for the patient; access second data associated with an estimated severity of a fall for the patient; and calculate a score based upon the first data and the second data.

These and other aspects and embodiments are described in detail below, in relation to the attached drawing figures.

BRIEF DESCRIPTION OF THE DRAWINGS

Figure 1 is a diagrammatic view of a patient support apparatus positioned in a room, with a control system of the patient support apparatus in electrical communication with other devices, controllers, and systems positioned inside and outside the room.

Figure 2 is a diagrammatic view of additional aspects of the room including a patient in the patient support apparatus, devices, controllers, and systems shown in Figure 1.

Figure 3 is a logical view of modules programmed to calculate an estimated likelihood of a fall for the patient of Figure 2.

Figure 4 is a timeline of activity for the patient of Figure 2.

Figure 5 is a diagrammatic representation of a display screen for a computing device of the caregiver showing the estimated likelihood of a fall for the patient of Figure 2.

Figure 6 is another diagrammatic representation of a display screen for a computing device of the caregiver showing the estimated likelihood of a fall for the patient of Figure 2.

Figure 7 is a method of calculating a score quantifying a likelihood of a fall for the patient of

Figure 2.

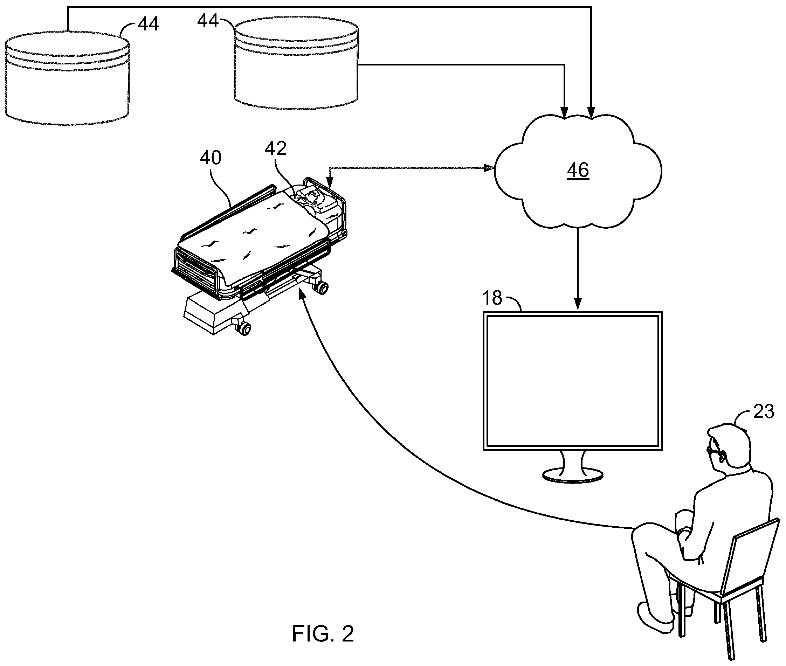

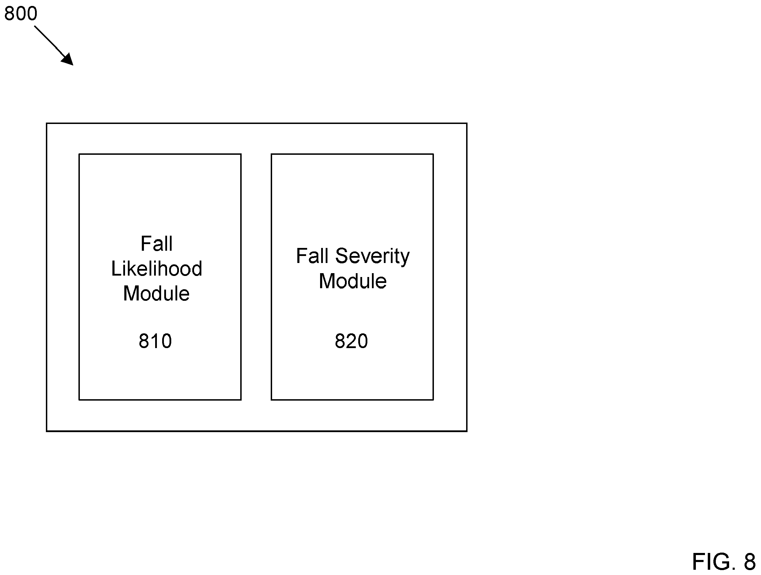

Figure 8 is a logical view of modules programmed to calculate an estimated severity of a fall for the patient of Figure 2.

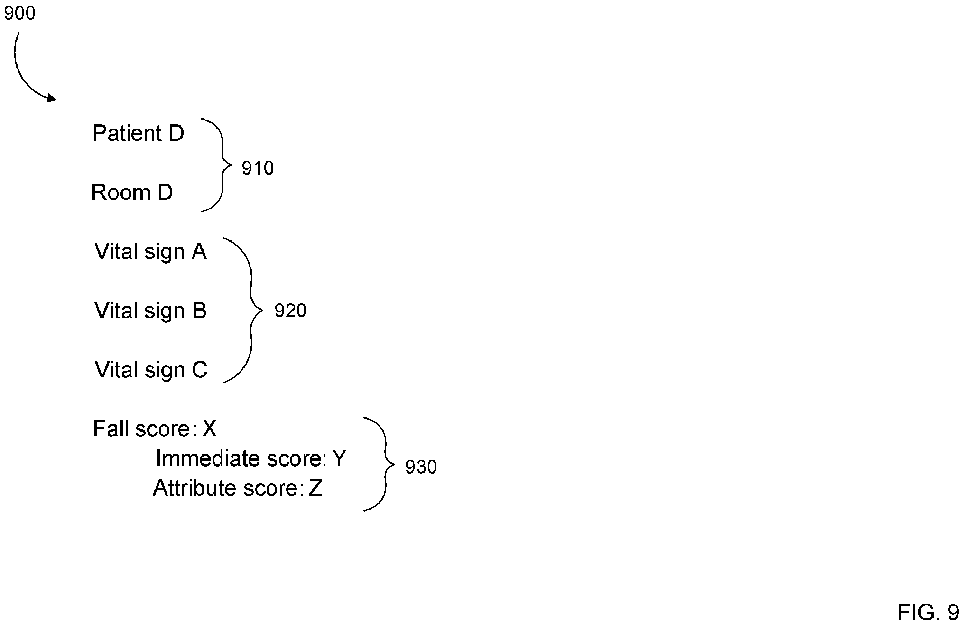

Figure 9 is a diagrammatic representation of a display screen for a computing device of the caregiver showing the estimated likelihood of a fall, severity of a fall, and overall risk of a fall for the patient of Figure 2.

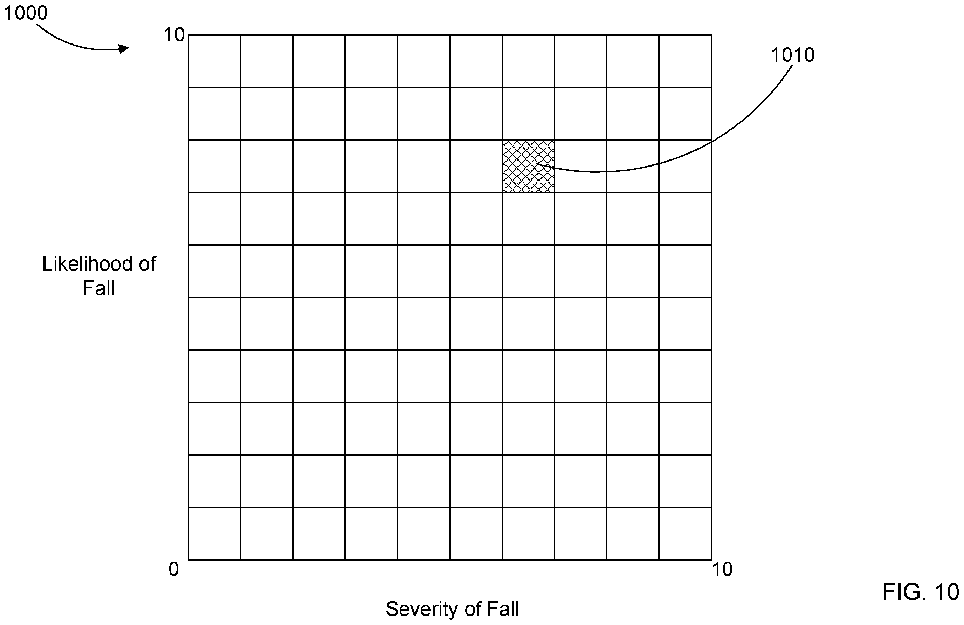

Figure 10 a diagrammatic graphical representation of a risk of a fall including the likelihood of a fall and the severity of a fall for the patient of Figure 2.

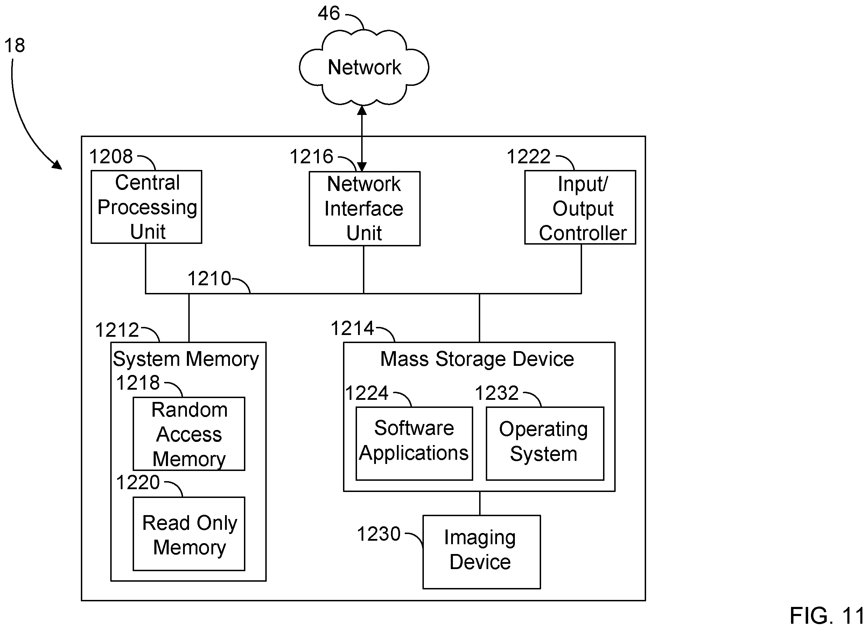

Figure 11 are example components of a computing device of Figure 2.

DETAILED DESCRIPTION

Various embodiments and advantages are explained more fully with reference to the nonlimiting examples that are described and illustrated in the accompanying drawings and detailed in the following description. The features illustrated in the drawings are not necessarily drawn to scale, and features of one embodiment may be employed with other embodiments, even if not explicitly stated herein.

The examples used herein are intended merely to facilitate an understanding of ways in which the claimed subject matter may be practiced and to enable those of skill in the art to practice the embodiments of the claimed subject matter described herein. Accordingly, the embodiments provided herein are merely illustrative and should not be construed as limiting the scope of the claimed subject matter, which is defined solely by the appended claims and applicable law. Moreover, like reference numerals may represent similar parts throughout the several views of the drawings.

The present disclosure describes improved methods and systems for estimating a likelihood of a fall for a patient. The methods and systems may assist caregivers in preventing unadvised, unmonitored bed exits and thus help prevent patient falls. The methods and systems may also allow for different patients to be monitored with varying levels of scrutiny, based at least in part on the needs of the individual patients, and may facilitate efficient and effective monitoring of multiple patients by a remote caregiver.

In the examples provided herein, the systems and methods utilize data from disparate sources and process that data efficiently to estimate a risk of a patient fall. In these examples, data from specialized devices and sensors is processed, and the estimate is calculated using (i) data associated with an immediate state of the patient and (ii) data associated with attributes of the patient. The potential severity of a fall can also be estimated and used to calculate an overall fall risk assessment.

This data is used in a practical application to provide the estimate of a patient fall to a caregiver. For example, the systems and methods can be programmed to use the data to generate a score indicating the likelihood of a patient fall. This score can be presented to the caregiver and can be used to provide alerting for the caregiver to mitigate the impact of such falls.

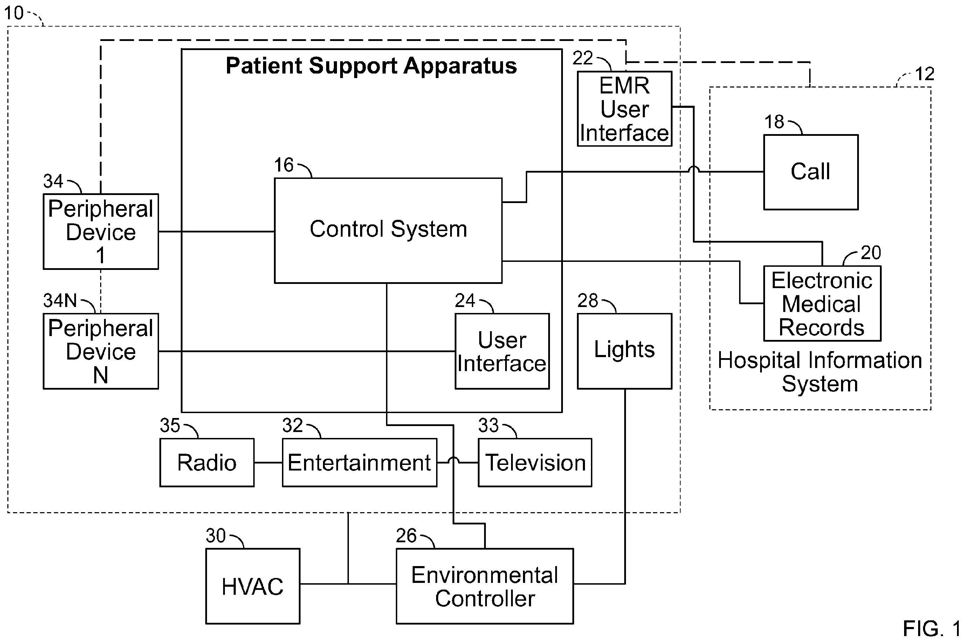

Figure 1 is a diagrammatic representation of the relationship between a patient support apparatus 14 positioned in a room 10 of a care facility and a hospital information system 12, according to one embodiment. This figure and details of other aspects and embodiments are described in U.S. Patent Application Pub. No. 2013/0246088, titled "Algorithm for Predicting and Mitigating Adverse Events," the full disclosure of which is hereby incorporated by reference. This exemplary embodiment is provided here as one example of an environment in which a patient support apparatus 14 may be positioned and used. It is not intended to be limiting but only exemplary.

In the illustrated embodiment, the hospital information system 12 includes a centralized caregiver call system 18 and a centralized electronic medical record system 20. Both the caregiver call system 18 and electronic medical records system 20 include information that is related to a patient support apparatus 14 and associated with the patient stored in memory as related records. The information related to the patient stored in memory in the caregiver call system 18 and electronic medical records system 20 is constantly updated as information is added to the electronic medical records system 20, and the caregiver call system 18 receives information related to the patient and the patient support apparatus 14.

The patient support apparatus 14 includes a control system 16 that is in communication with the caregiver call system 18 through a network (e.g., network 46 shown in Figures 2 and 8). The control system 16 includes a user interface 24 that is used by the patient supported on the patient support apparatus 14 or a caregiver to provide inputs to the control system 16 or display outputs from the control system 16.

As shown diagrammatically in Figure 1, the electronic medical records system 20 is in electrical communication through the network 46 with a user interface 22 positioned in the room 10 and accessible by a caregiver to input patient information and enter orders while the caregiver is in the room 10. The user interface 22 may be a personal computing device or a dedicated peripheral computing device. It should be understood that other user interfaces may be used throughout a facility to interface with the hospital information system 12, and specifically the electronic medical records system 20.

In the illustrative embodiment of Figure 1, the user interface 24 is positioned on the patient support apparatus 14 and may be used by caregiver to access the electronic medical records system 20 through the control system 16 of the patient support apparatus 14, which is in direct communication with the electronic medical records system 20 and acts as a peripheral device to the electronic medical records system 20.

The control system 16 is also in communication with an environmental systems controller 26, which provides an interface between the patient support apparatus 14 and various environmental systems including lights 28, heating-ventilating-air-conditioning system 30, and entertainment devices 32 such as a television 33 or radio 35, for example. The environmental systems controller 26 provides information to the control system 16 and acts on instructions from the control system 16 to modify operation of the environmental systems. Some of the information provided by the environmental systems controller 26 is stored in memory associated with the environmental systems controller 26. The information provided by the environmental systems controller 26 is updated as operating parameters of the environmental systems change.

The control system 16 may also be in communication with one or more peripheral devices 34 positioned in the room 10. The peripheral devices 34 each perform a therapy or diagnostic function. For example, the peripheral device 34 may be a ventilator, heart monitor, blood pressure monitor, infusion device, blood oxygen monitor, sequential compression device, high-frequency chest wall oscillation device, and/or another standalone diagnostic or therapeutic device.

In one example, one of the peripheral devices 34 is a patient monitoring device, such as a CONNEX® Vital Signs Monitor provided by Welch Allyn, Inc. of New York. In this example, one or more wireless and/or wired sensors are associated with the patient, and vital signs data from those sensors is communicated to the patient monitoring device. The data can be processed by the patient monitoring device and/or communicated to the caregiver call system 18 and electronic medical records system 20.

Information used by the control system 16 may be stored in memory associated with a peripheral device 34, including the therapy parameters or current operating conditions of the peripheral device 34. In addition, diagnostic values such as a heart rate, blood pressure, or other diagnostic values may be stored in memory associated with the peripheral device. In some cases, the peripheral devices 34 may communicate to the controller 26 via a network connection such as a controller area network (CAN) and information stored on a controller of the device 34 may be accessible by the controller 26.

In other cases, the information may be stored by the hospital information system 12. In still other cases, the peripheral devices 34 may communicate with the controller 26 and the controller 26 may store information related to the operator of the peripheral device(s) 34 in memory of the controller 26. As illustrated in Figure 1, any number of peripheral devices 34 may be in communication with the patient support apparatus 14. It should be understood that the peripheral devices 34 may be in direct communication with the hospital information system 12 without being connected through the patient support apparatus 14.

The caregiver call system 18 generates alarms and notifies caregivers of alarm conditions based on signals from the control system 16 of the patient support apparatus 14 and information from the electronic medical records system 20. It is also known in the art for the patient support apparatus 14 to provide a communication link, such as audio or video communications, between a patient supported on the patient support apparatus 14 and a caregiver positioned at the central caregiver call system 18. In one example, the caregiver call system 18 is a CONNEX® Central Station provided by Welch Allyn, Inc. of New York. Other configurations are possible.

It is also known for caregivers to carry communication badges that include telephone or other voice communication capability, with the badges providing a direct communication between the caregiver and the central caregiver call station 18 or patient, such as the system disclosed in U.S. Pat. No. 7,746,218 titled "Configurable System for Alerting Caregivers," incorporated by reference herein. The caregiver call system and/or communication badges may facilitate direct communication between a caregiver and a patient positioned on any patient support apparatus is 14 throughout a care facility. In this way, the caregiver call system 18 acts as a dispatch system to provide instructions to caregivers when various conditions warrant the intervention of the caregiver either to make adjustments to equipment or to respond to the needs of a particular patient.

As mentioned above, various embodiments described herein provide methods and systems for monitoring a patient in a hospital bed or other patient support apparatus to estimate a likelihood of a fall for a patient. For clarity, the patient support apparatus referenced above will be referred to below as a "bed". In alternative embodiments, however, the patient support apparatus may be a chair, a recliner, or any other patient support apparatus. The bed may be located in the patient's home or in any patient care facility, such as but not limited to a hospital, clinic, surgi-center, nursing home, skilled nursing facility, or the like.

Figure 2 is a simplified, diagrammatic representation of additional aspects of the room 10 of a care facility and the hospital information system 12 of Figure 1, according to one embodiment. In the illustrated embodiment, a patient 42 lies on a bed 40. Multiple databases 44 house data collected from various sources in the room 10 and hospital information system 12 and are accessible through the network 46.

The caregiver call system 18 includes one or more computing devices that use algorithms to process the data from the databases 44 to provide a caregiver 23 with an estimate of likelihood of a fall for the patient. In this example, the caregiver 23 can be located remotely from the patient 42, such as at the caregiver call system 18.

When the caregiver call system 18 estimates that a likelihood for a fall exceeds a threshold, the caregiver call system 18 can provide an indication to and/or alert the caregiver 23. This alert may be delivered in any suitable form, such as a text message, pager message, email, or other form of alert information, such as a message on a display device associated with the caregiver call system 18. Alternatively or additionally, an alert or reminder can be provided to the patient 42 to stay in bed, to be careful when exiting the bed, or the like. The alert or reminder may be provided, for example, via a voice command delivered over a microphone/intercom system in the patient's room.

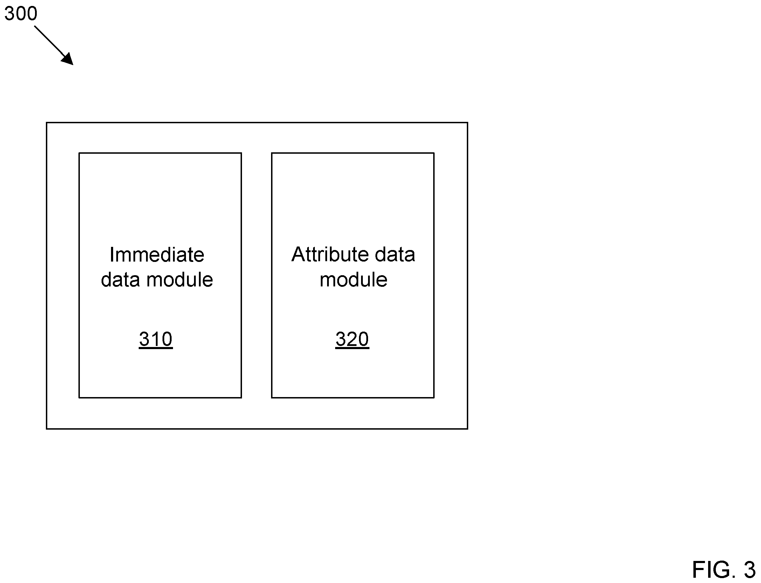

Figure 3 shows logic 300 that is implemented by one or more computing devices. Specifically, the one or more computing devices include at least one processor that executes instructions stored in memory to implement the logic 300. In this example, the computing devices include the caregiver call system 18. In other examples, some or all of the logic can also be implemented in other computing devices, such as one of the peripheral devices 34, the electronic medical records system 20, and/or one or more computing devices associated with a server or cloud-computing platform.

In this example, the logic 300 includes an immediate data module 310 and an attribute data module 320. In a general sense, the immediate data module 310 and the attribute data module 320 are used to estimate a likelihood of a fall for a given patient. This likelihood can be used, as described herein, to mitigate such falls through alerting and other actions.

The immediate data module 310 develops an immediate risk model that generally addresses the immediate risk of a patient falling based on the present actions of the patient and the environment surrounding the patient (i.e., taking place in real-time / acute). For example, this immediate risk model considers what the patient is doing in real time, such as the perceived urgency of attempting to leave the bed, leaving the bed at all, any change of medication, and even contextual information, such as if it is the middle of the night or the rails on the bed are up or down. This can be used by the caregivers to determine the likelihood that a patient is going to fall soon (e.g., in the next 30 seconds) and if they need to intervene immediately.

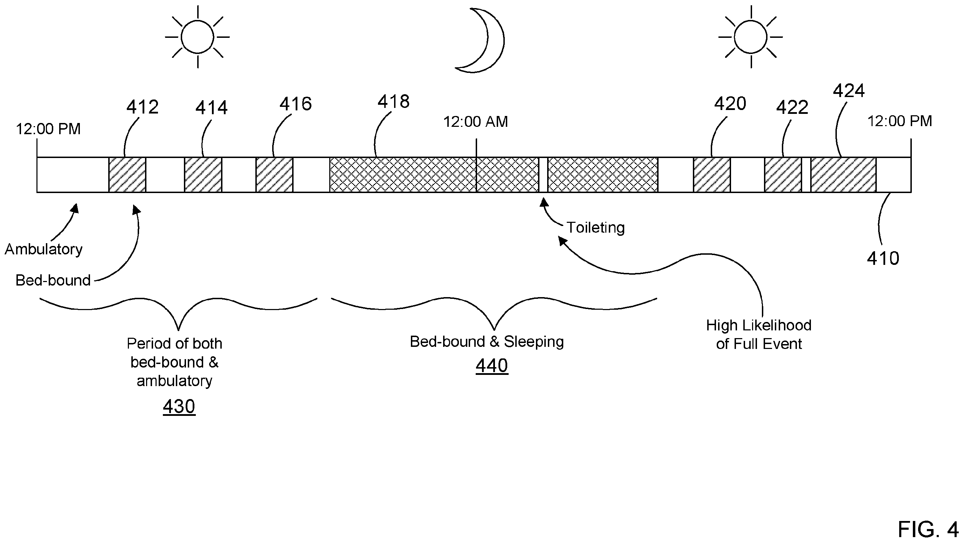

For example, Figure 4 illustrates a timeline 400 associated with activity for a given patient. The timeline 400 quantifies an awake period 430 when a patient is awake and a sleeping period 440 when the patient is sleeping or otherwise confined to the bed. Activities associated with the patient at the various times are indicated on the timeline 400, such as bed-bound activities 412, 414, 415, 418, 420, 422, and 424 like relaxing and sleeping. Other times on the timeline 400 indicate when the patient is active or otherwise, out of the bed (i.e., ambulatory). Such activities that can be tracked include walking, toileting, etc.

These activities can be captured manually and inputted into the system (e.g., by the user interface 22). Alternatively, one or more sensors can be used to determine a state of the patient, such as an activity sensor that tracks the patient's orientation and movement (e.g., lying down, sitting up, walking, etc.). In another example, audio or video can be used to assess the state of the patient. An example of a system that uses video to assess patient fall risk is provided in U.S. Patent Application No. 15/866972 filed on January 10, 2018, the entirety of which is hereby incorporated by reference.

Such information can be stored, for example, in the databases 44 and/or the electronic medical records system 20. In this manner, the patient's current activity status is provided as a component to the immediate data module 310 to form the immediate risk model.

More specifically, the immediate risk model can be formed by the immediate data module 310 using data from a plurality of inputs. This data can include activity at a given period of time (e.g., toileting during sleeping hours), a medication change, acute motion detected by the patient, etc. This information is used by the immediate data module 310 to create the immediate risk model score, as provided below:

$$\text{immediate risk model score} = \text{data1} \times \text{weight1} + \text{data2} \times \text{weight2} + \dots \text{dataN} \times \text{weightN}$$

In this example, the immediate risk model score is a numerical quantification of the likelihood of an immediate fall as calculated by the immediate data module 310. Each relevant piece of data is weighted and added to create the score. For example, the acute movement of the patient can be weighted more highly than a change in medication. The immediate risk model score can be used as described further below to estimate a likelihood of a fall for a patient and/or provide alerting to a caregiver to mitigate the fall.

Referring again to Figure 3, the attribute data module 320 develops an attribute risk model that addresses a general indicator of the risk of the patient falling due to overall risk factors that are developed over time. The risk factors include bibliographic / demographic information associated with the patient, such as a history of falling, age, frequency or urgency of urination, etc. Other risk factors can include the type of medication taken, the procedures under which the patient has gone, a gait analysis, etc. In other words, the attribute risk model can inform caregivers about the amount of vigilance and/or interventions that should take place to optimize safety for the patient.

As illustrated in Figure 4, during the course of the first few days in bed, there will be periods of time when the patient is in bed and when the patient is out of the bed, ambulating. Over time, bed-based data (load beams, pressure sensors, for instance) is used to determine factors such as mobility (in-bed motion), and levels of consciousness for the patient can be gathered. In the period that the patient is ambulating, patient-worn sensors (e.g., accelerometers, etc.) can be used to gather more data on the patient about how they are moving. Further sensors, such as gait-detection, can be used as a way of determining fall risk.

This data is stored over time (e.g., in the databases 44 and/or the electronic medical records system 20) as the sensors collect information about the patient. The data is then used by the attribute data module 320 to create the attribute risk model score, as provided below:

$$\text{attribute risk model score} = \text{data1} \times \text{weight1} + \text{data2} \times \text{weight2} + \dots \text{dataN} \times \text{weightN}$$

In this example, the attribute risk model score is a numerical quantification of the likelihood of a fall as calculated by the attribute data module 310 based upon the attributes of the patient collected over time. Each relevant piece of data is weighted and added to create the score. For example, the poor gait of the patient can be weighted more highly than motion of the patient in the bed over time. The attribute risk model score can be used as described further below to estimate a likelihood of a fall for a patient and/or provide alerting to a caregiver to mitigate the fall.

The scores calculated by the immediate data module 310 and the attribute data module 320 can be combined to provide a more complete score that estimates a likelihood of a fall for the patient, as provided below:

$$\text{fall score} = \text{immediate risk model score} + \text{attribute risk model score}$$

This combined "fall score" can be presented to the caregiver and/or used for alerting purposes. The benefit of the fall score is that the score accounts for both attributes associated with the patient over time and immediate actions and conditions surrounding the patient that may indicate a likelihood for a fall. This overall fall score provides a better indication of the likelihood of a fall for the patient, thereby minimizing false alerts will still providing meaningful and optimized fall protection.

In some examples, the fall score is updated on a periodic basis. This can be near-real-time, such as once per second, or based upon a greater period, such as once every five seconds, once a minute, etc. The fall score can be compared to a threshold value. This threshold value can be specific to the patient (e.g., based upon the patient's prior history) or can be general to a population associated with the patient (e.g., age, sex, etc.). If the fall score exceeds a threshold, the caregiver can be alerted as indicated herein regarding a likelihood of a fall for the patient.

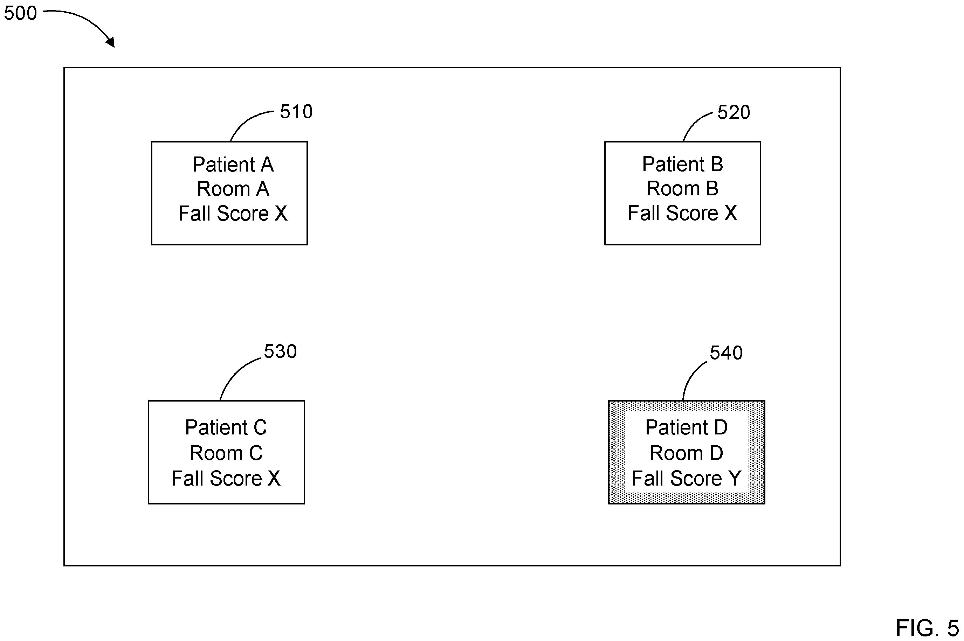

Referring to Figure 5, an example user interface 500 for the caregiver call system 18 is provided. In this example, the interface 500 includes entries for a plurality of patients 510, 520, 530, 540. For each patient, the interface 500 provides bibliographic information (patient name and location) and the fall score calculated for each patient, as identified above. In addition, the fall score for the patient 540 exceeds the threshold for that patient, so the interface 500 provides an alert by highlighting the patient 540 on the interface 500. The alerting can include highlighting, flashing, audio, and/or other indications to alert the caregiver.

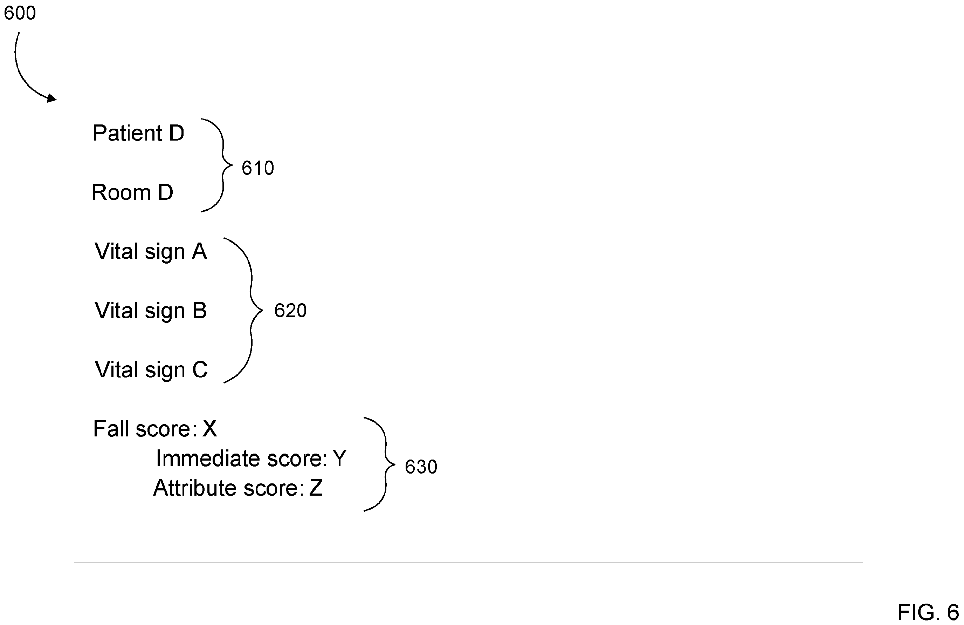

Figure 6 shows another example user interface 600 for the caregiver call system 18. This interface 600 provides more information for a particular patient, such as the patient 540. The caregiver can access the interface 600 by selecting the patient 540 on the interface 500.

The information provided on the interface 600 can include bibliographic information 610, like patient identifier and room, as well as vital sign information 620, like heart rate, blood pressure, and oxygen saturation. In addition, the interface 600 provides fall information 630, including the immediate risk model score Y, the attribute risk model score Z, and the combined fall score X. This allows the caregiver to make a more complete assessment of the likelihood of a fall for the patient 540.

In some examples, the alerting information provided on the interface 600 and/or alerts to the appropriate caregiver(s) can provide additional contextual information, such as how to intervene to mitigate the fall risk. In such examples, in addition to providing the fall score, a suggested action for the caregiver can be provided. For example, an action such as, "Prevent fall for patient X in room Y" can be provided as part of the alert. In other examples, even more context can be provided, such as, "Likelihood of fall is Z percent, intervene now for patient X in room Y." In yet other examples, certain aspects can be highlighted, such as a reason that the fall score has increased: "Patient X has left bed and is compromised because of current medication, intervene now in room Y." Other examples are possible.

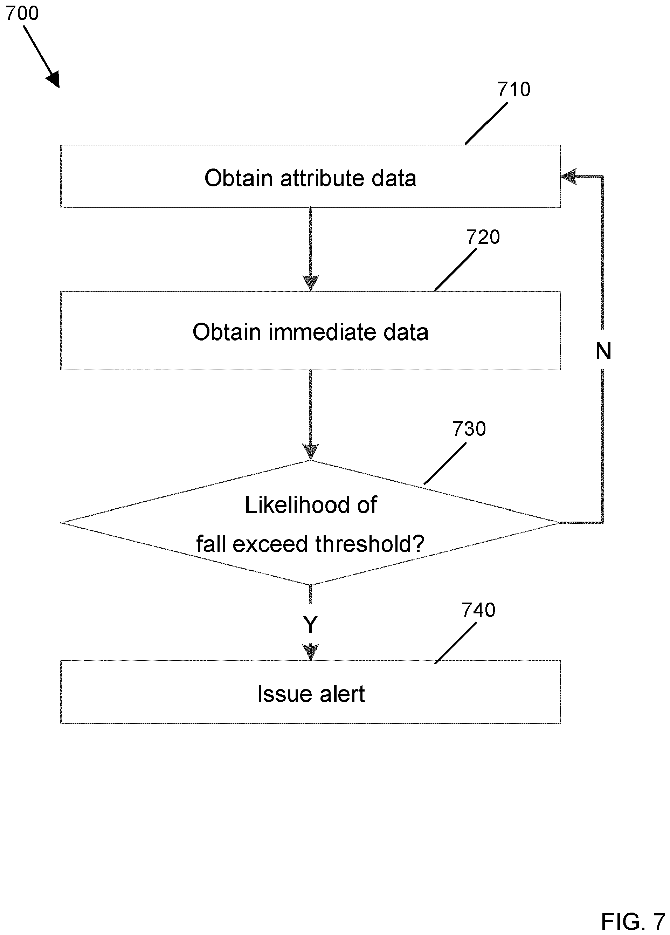

Figure 7 provides an example method 700 for assessing the likelihood of a fall for a particular patient. The method 700 can be implemented, for example, by the caregiver call system 18 as described above.

At operation 710, the attribute data is obtained (e.g., by the attribute data module 320 over an extended period). Next, at operation 720, the immediate data is obtained (e.g., by the immediate data module 310). Next, at operation 730, an assessment of the likelihood of a fall (e.g., by calculating the fall score) is performed and compared to a threshold. If the likelihood exceeds a threshold, control is passed to operation 740 for alerting. If not, control is passed back to operation 710.

In some examples, the attribute data is only accessed periodically, since the attribute data does not change as rapidly as the immediate data. For example, the attribute data can be accessed and an updated attribute risk model score is calculated at a longer interval (e.g., once every five minutes, ten minutes, 30 minutes, 1 hour, etc.) than the updating of the immediate risk model score (e.g., once every second, once every five seconds, etc.).

Figure 8 shows logic 800 that is implemented by one or more computing devices. Specifically, the one or more computing devices include at least one processor that executes instructions stored in memory to implement the logic 800. In this example, the computing devices include the caregiver call system 18. In other examples, some or all of the logic can also be implemented in other computing devices, such as one of the peripheral devices 34, the electronic medical records system 20, and/or one or more computing devices associated with a server or cloud-computing platform.

In this example, the logic 800 includes a fall likelihood module 810 and a fall severity module 820. In a general sense, the fall likelihood module 810 estimates a likelihood of a fall for a given patient, and the fall severity module 820 estimates a potential severity of a fall for a given patient. This information can be used, as described herein, to mitigate such falls through alerting and other actions.

The fall likelihood module 810 accesses data to estimate the likelihood that a fall will occur. In this example, the fall likelihood module 810 can use the fall score calculated above, based upon the immediate risk model and the attribute risk model. The fall score module 810 can also use other methods for calculating a likelihood of a fall. For example, in another embodiment, the fall likelihood module can use only the immediate risk model to estimate the likelihood of a fall. Other configurations are possible.

The fall severity module 820 estimates a potential severity of a fall. This severity is separate from whether or not a fall is imminent. In other words, the fall severity module 820 attempts to quantify the potential severity of a fall should one occur.

In some examples, the fall severity module 820 estimates the potential severity based upon certain demographics associated with the patient. For example, the fall severity module 820 can examine such characteristics as a patient's age, gender, size (height and/or weight) to ascertain the potential severity of a fall. In a simple example, older patients can be assumed to sustain more damage from a fall as opposed to younger patients and therefore would receive a higher potential severity.

The fall severity module 820 can also examine the health state of the patient. In some instances, a patient with osteoporosis (i.e., bone brittleness), low vitamin D levels, or heart disease can sustain greater damage from a fall. Other aspects of health, such as myopia or balance disorders, can also be examined. Further, mental health can also be an indicator, such as dementia. In addition, drugs can also be a factor. For example, some drugs create a higher likelihood of bruising and/or bleeding, which can impact the potential severity of a fall.

In an alternative embodiment, the fall severity module 820 can also account for an activity state of the patient. For example, the likely severity of a fall could be impacted based upon the location and activity level of the patient. A hospital room may be a safer environment for a fall than transitioning between rooms or outside on pavement. A height of the patient can also impact a likely severity - a fall from a sitting position may be less severe than a fall from an elevated position, such as during walking or positioned on a bed. In addition, the speed at which a patient is traveling could impact severity, with a faster pace making a greater severity more likely.

In some embodiments, the fall severity module 820 can use data from large populations of patients to generate correlations between the patient characteristics (e.g., demographics and/or health state) and potential fall severities. For example, the fall severity module 820 can examine fall statistics from large populations of patients to understand correlations between different characteristics and the severity of a fall. An example of such a correlation could be that patients with dementia typically sustain greater trauma from a fall than a control group of patients without dementia, possibly because the dementia reduces a patient's natural ability to catch one's self during a fall. Many other possible correlations and examples are possible. Such information can be stored, for example, in the databases 44 for access by the fall severity module 820.

The models created by the fall likelihood module 810 and the fall severity module 820 can be used to create an overall fall risk score (or injury score) for a patient. This overall fall risk score, as provided below, quantifies the potential for an injury should a fall occur:

$$\text{overall fall risk score} = \text{fall likelihood model score} \times \text{fall severity model score}$$

In this example, a fall severity model score greater than 1 will increase the overall fall risk score, while a fall severity model score of less than 1 will decrease the overall fall risk score. This information can be depicted in various manners to the caregiver, as provided in more detail below.

Figure 9 shows another example user interface 900 for the caregiver call system 18. This interface 900 provides information about the overall fall risk for a particular patient (e.g., patient 540).

The information provided on the interface 900 can include bibliographic information 910, like patient identifier and room, as well as vital sign information 920, like heart rate, blood pressure, and oxygen saturation. In addition, the interface 900 provides fall information 930, including a fall likelihood score Y, a fall severity score Z, and the overall fall risk score X. This allows the caregiver to make a more complete assessment of the risk of a fall for the patient 540.

In some examples, the alerting information provided on the interface 900 and/or alerts to the appropriate caregiver(s) can provide additional contextual information, such as how to intervene to mitigate the fall risk. In such examples, in addition to providing the overall fall risk score, the alert can provide both the likelihood of a fall and potential severity of a fall: "Patient X has a likelihood of falling, but the potential severity of the fall is low." Other examples are possible.

In some embodiments, the alerting can be configured based upon the needs of a specific hospital or caregiver. For example, the threshold for alerting can be made configurable, so that the caregiver can increase or decrease the sensitivity of the system as desired. In addition, alerting can be provided, both on an absolute threshold as well as on a percentage change in the fall risk level. In other words, in some embodiments, an increase in the fall risk level of a certain percentage (e.g., 10 percent, 25 percent, etc.) can trigger an alert even when the fall risk level remains below the absolute threshold.

Examples of other configurable aspects for alerts include who, when, or where notifications or alerts are sent locally/remotely. The types of alerts can also be configurable, such as visual and/or audible (e.g., annunciated to the caregiver).

Referring to Figure 10, an example graphical representation 1000 of an overall fall risk score 1010 is shown. In this graphical representation 1000, the horizontal axis depicts a potential severity of the fall (from 0 to 10), and the vertical axis depicts the likelihood of a fall (from 0 to 10). The overall fall risk score 1010 for the patient is depicted on the graphical representation 1000 at the appropriate position.

This depiction is similar to a "heat map" or other matrix. A lower position on the graph indicates less likelihood of a fall, and the likelihood increases as the position increases vertically. On a similar note, as the position of the patient moves from the vertical axis towards the right, the potential severity of a fall increases. The graphical representation 1000 can help the caregiver to easily visualize the risks associated with a fall for a patient.

In one example, the graphical representation 1000 can be depicted on the interface 900 for the patient. The caregiver can glance at the graphical representation 1000 and note the placement of the overall fall risk score 1010 relative to the graph and act should the likelihood of a fall, the potential severity of a fall, and/or the combined risk reach a critical level. The overall fall risk score 1010 can also be coded, such as with colors, to indicate an increase in risk. As the overall fall risk score 1010 moves upwards and to the right (indicating greater likelihood and severity), the overall fall risk score 1010 can change from a first color, such as green, to a second color, such as yellow or red to indicate increased risk. Other configurations are possible.

In yet another example, the graphical representation 1000 can be modified to show further information. For instance, the likelihood of a fall can be broken into separate components for depicting the immediate risk of fall and attribute risk of fall. This can result in another dimension for the graphical representation 1000, or the caregiver can select which items to display. For example, the caregiver can decide to just show the items associated with the likelihood of a fall (e.g., with the immediate risk of fall on the horizontal axis and the attribute risk of fall on the vertical axis) or show both the likelihood of a fall with the potential severity, as depicted in Figure 10.

There can be various advantages to incorporating a potential severity of a fall into the overall fall risk estimation. Specifically, the severity can be an indicator of when intervention is necessary, thereby decreasing false alarms and better prioritizing limited caregiver resources. For example, a young, relatively healthy patient can be much less likely to be severely injured during a fall, so the overall risk of a fall can be diminished. Alternatively, an older patient with osteoporosis who is walking may be much more likely to sustain a severe fall, thereby increasing the overall risk of a fall.

Figure 11 illustrates example physical components of a computing device, such as the computing device or devices associated with the caregiver call system 18. As illustrated, the device includes at least one processor or central processing unit ("CPU") 1208, a system memory 1212, and a system bus 1210 that couples the system memory 1212 to the CPU 1208. The system memory 1212 includes a random access memory ("RAM") 1218 and a read-only memory ("ROM") 1220. A basic input/output system containing the basic routines that help to transfer information between elements within the device, such as during startup, is stored in the ROM 1220. The device further includes a mass storage device 1214. The mass storage device 1214 is able to store software instructions and data. The central processing unit 1208 is an example of a processing device.

The mass storage device 1214 is connected to the CPU 1208 through a mass storage controller (not shown) connected to the bus 1210. The mass storage device 1214 and its associated computer-readable data storage media provide non-volatile, non-transitory storage for the device. Although the description of computer-readable data storage media contained herein refers to a mass storage device, such as a hard disk or CD-ROM drive, it should be appreciated by those skilled in the art that computer-readable data storage media can be any available non-transitory, physical device or article of manufacture from which the device can read data and/or instructions. The mass storage device 1214 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, CD-ROMs, digital versatile discs ("DVDs"), other optical storage media, magnetic cassettes, magnetic tape, magnetic disk storage or other magnetic storage devices, or any other medium which can be used to store the desired information and which can be accessed by the device.

According to various embodiments, the device may operate in a networked environment using logical connections to remote network devices through the network 46, such as a local network, the Internet, or another type of network. The device connects to the network 46 through a network interface unit 1216 connected to the bus 1210. The network interface unit 1216 may also be utilized to connect to other types of networks and remote computing systems. The device also includes an input/output controller 1222 for receiving and processing input from a number of other devices, including a camera, a keyboard, a mouse, a touch user interface display screen, or another type of input device. Similarly, the input/output controller 1222 may provide output to a touch user interface display screen, a printer, or other type of output device.

The device also includes an imaging device 1230, such as a camera that is configured to capture still or moving images (i.e., video). The camera can be configured to capture high resolution images or video (e.g., 100-200+ fps) that can be used to conduct one or more of the analyses described herein.

As mentioned above, the mass storage device 1214 and the RAM 1218 of the device can store software instructions and data. The software instructions include an operating system 1232 suitable for controlling the operation of the device. The mass storage device 1214 and/or the RAM 1218 also store software instructions, that when executed by the CPU 1208, cause the device to provide the functionality of the device discussed in this document.

The use of the terms "a" and "an" and "the" and similar referents in the context of describing the subject matter (particularly in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. Furthermore, the foregoing description is for the purpose of illustration only, and not for the purpose of limitation, as the scope of protection sought is defined by the claims as set forth hereinafter together with any equivalents thereof entitled to.

The use of any and all examples, or exemplary language (e.g., "such as") provided herein, is intended merely to better illustrate the subject matter and does not pose a limitation on the scope of the subject matter unless otherwise claimed. The use of the term "based on" and other like phrases indicating a condition for bringing about a result, both in the claims and in the written description, is not intended to foreclose any other conditions that bring about that result.

**Claims**

1. A system for estimating an injury risk relating to a fall for a patient, the system comprising:

   at least one processor; and
   memory encoding instructions which, when executed by the at least one processor, cause the at least one processor to:

   access first data associated with an estimated likelihood of a fall for the patient;
   access second data associated with an estimated severity of a fall for the patient; and
   calculate a score based upon the first data and the second data.

2. The system of claim 1, wherein the memory encodes further instructions which, when executed by the at least one processor, cause the at least one processor to:

   access demographic data associated with the patient;
   access health state data associated with the patient; and
   use the demographic data and the health state data to provide the estimated severity of a fall for the patient.

3. The system of claim 2, wherein the memory encodes further instructions which, when executed by the at least one processor, cause the at least one processor to:

   access activity data for the patient; and
   use the demographic data, the health state data, and the activity data to provide the estimated severity of a fall for the patient.

4. The system of claim 1, 2 or 3, wherein the memory encodes further instructions which, when executed by the at least one processor, cause the at least one processor to:

   develop an immediate risk of fall model using the first data;

develop an attribute risk of fall model using the first data; and
use the immediate risk of fall model and the attribute risk of fall model to provide the estimated likelihood of a fall for the patient.

5. The system of any of the preceding claims, wherein the memory encodes further instructions which, when executed by the at least one processor, cause the at least one processor to provide an alert when the score exceeds a threshold.

6. The system of claim 5, wherein the alert provides additional contextual information about the patient, and/or wherein the alert is configurable by the caregiver.

7. The system of claim 6, wherein the memory encodes further instructions which, when executed by the at least one processor, cause the at least one processor to allow the caregiver to configure alerting based upon the threshold or a change in the score, and/or
wherein the memory encodes further instructions which, when executed by the at least one processor, cause the at least one processor to allow the caregiver to configure alerting based upon who is alerted, when alerts are sent, and how alerts are delivered.

8. The system of any of the preceding claims, wherein the memory encodes further instructions which, when executed by the at least one processor, cause the at least one processor to generate a user interface to display at least one of: the score; a fall likelihood score; and a fall severity score.

9. A method for estimating an injury risk relating to a fall for a patient, the method comprising:

accessing first data associated with an estimated likelihood of a fall for the patient;
accessing second data associated with an estimated severity of a fall for the patient; and
calculating a score based upon the first data and the second data.

10. The method of claim 9, further comprising:

accessing demographic data associated with the patient;
accessing health state data associated with the patient; and
using the demographic data and the health state data to provide the estimated severity of a fall for the patient.

11. The method of claim 10, further comprising:

accessing activity data for the patient; and
using the demographic data, the health state data, and the activity data to provide the estimated severity of a fall for the patient.

12. The method of claim 9, 10 or 11, further comprising:

developing an immediate risk of fall model using the first data;
developing an attribute risk of fall model using the first data; and
using the immediate risk of fall model and the attribute risk of fall model to provide the estimated likelihood of a fall for the patient.

13. The method of any of claims 9 to 12, further comprising providing an alert when the score exceeds a threshold, preferably wherein the alert provides additional contextual information about the patient.

14. The method of claim 13, further comprising allowing the caregiver to configure alerting based upon the threshold or a change in the score, and/or further comprising allowing the caregiver to configure alerting based upon who is alerted, when alerts are sent, and how alerts are delivered.

15. The method of any of claims 9 to 14, further comprising generating a user interface to display at least one of: the score; a fall likelihood score; and a fall severity score.

FIG. 1

FIG. 2

EP 3 709 308 A1

EP 3 709 308 A1

300

```
┌─────────────────────────────────────────┐
│                                         │
│   ┌──────────────┐   ┌──────────────┐   │
│   │              │   │              │   │
│   │  Immediate   │   │ Attribute data│  │
│   │ data module  │   │    module    │   │
│   │              │   │              │   │
│   │              │   │              │   │
│   │     310      │   │     320      │   │
│   │              │   │              │   │
│   └──────────────┘   └──────────────┘   │
│                                         │
└─────────────────────────────────────────┘
```

FIG. 3

FIG. 4

EP 3 709 308 A1

500

510

Patient A
Room A
Fall Score X

520

Patient B
Room B
Fall Score X

530

Patient C
Room C
Fall Score X

540

Patient D
Room D
Fall Score Y

FIG. 5

FIG. 6

700

710

Obtain attribute data

720

Obtain immediate data

N

730

Likelihood of

fall exceed threshold?

Y

740

Issue alert

FIG. 7

EP 3 709 308 A1

800

Fall
Likelihood
Module

810

Fall Severity
Module

820

FIG. 8

900

Patient D
Room D          } 910

Vital sign A
Vital sign B    } 920
Vital sign C

Fall score: X
    Immediate score: Y    } 930
    Attribute score: Z

EP 3 709 308 A1

FIG. 9

FIG. 10

EP 3 709 308 A1

FIG. 11

Europäisches
Patentamt

European
Patent Office

Office européen
des brevets

## EUROPEAN SEARCH REPORT

Application Number

EP 20 16 2929

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/357930 A1 (SINGH HARPREET [US] ET AL) 8 December 2016 (2016-12-08)<br>* The whole document, in particular:<br>Paragraphs [0006], [0007], [0018] - [0022], [0023], [0031]; claims 1 - 4, 9. * | 1-15 | INV.<br>G16H50/30<br><br>ADD.<br>A61B5/00 |
| X | US 2014/214440 A1 (YOUNG DAVID CORT [US] ET AL) 31 July 2014 (2014-07-31)<br>* The whole document, in particular:<br>Paragraphs [0005] - [0009], [0114] - [0124], [0195] - [0241]; Figures 2, 3, 7A - 7D. * | 1-15 | |
| X | US 9 875 450 B1 (HENDRICK III JAMES ROBERT [US] ET AL) 23 January 2018 (2018-01-23)<br>* The whole document, in particular:<br>Columns 11 - 17. * | 1-15 | |

TECHNICAL FIELDS
SEARCHED      (IPC)

G16H
A61B

The present search report has been drawn up for all claims

| Place of search | Date of completion of the search | Examiner |
|---|---|---|
| Munich | 8 July 2020 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 16 2929

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2020

| Patent document cited in search report | | Publication date | Patent family member(s) | | Publication date |
|---|---|---|---|---|---|
| US 2016357930 | A1 | 08-12-2016 | US | 2016357930 A1 | 08-12-2016 |
| | | | US | 2020152334 A1 | 14-05-2020 |
| US 2014214440 | A1 | 31-07-2014 | US | 2014214439 A1 | 31-07-2014 |
| | | | US | 2014214440 A1 | 31-07-2014 |
| | | | US | 2014214441 A1 | 31-07-2014 |
| | | | WO | 2014117149 A1 | 31-07-2014 |
| | | | WO | 2014117150 A1 | 31-07-2014 |
| | | | WO | 2014117151 A1 | 31-07-2014 |
| US 9875450 | B1 | 23-01-2018 | US | 9257029 B1 | 09-02-2016 |
| | | | US | 9875450 B1 | 23-01-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130246088
- US 7746218 B

- US 86697218